# EUROPÄISCHE PATENTSCHRIFT

(11) **EP 1 222 220 B1**
(45) Veröffentlichungstag und Bekanntmachung des Hinweises auf die Patenterteilung: **13.08.2003**
(21) Anmeldenummer: 00960583.3
(22) Anmeldetag: 01.09.2000
(51) Int. Cl.: C08G 18/08, C08G 18/12, C08F 290/14, A61K 6/09, C08G 18/66

(54) **FÜLLSTOFF FÜR KUNSTSTOFF-FORMULIERUNGEN AUF POLYURETHANBASIS**
FILLER FOR PLASTIC FORMULATIONS BASED ON POLYURETHANE
CHARGE POUR FORMULATIONS DE MATIERE PLASTIQUE A BASE DE POLYURETHANE

(30) Priorität: 02.09.1999 DE 19941738
(43) Veröffentlichungstag der Anmeldung: 17.07.2002
(73) Patentinhaber: 3M Espe AG, 82229 Seefeld (DE)
(72) Erfinder: HECHT, Reinhold, D-86916 Kaufering (DE); GANGNUS, Bernd, D-82346 Andechs (DE); LECHNER, Günther, D-82237 Wörthsee (DE)
(74) Vertreter: Freiherr von Wittgenstein, Arved, Dr.
(86) Internationale Anmeldenummer: EP0008569
(87) Internationale Veröffentlichungsnummer: WO01018085

(56) Entgegenhaltungen:
- EP-A- 0 872 502
- US-A- 4 553 940
- US-A- 5 135 963
- US-A- 5 684 081

## Beschreibung

Die Erfindung betrifft Füllstoffe für Kunststoff-Formulierungen auf Polyurethanbasis. Insbesondere betrifft die Erfindung organische, vernetzte, reaktive und strahlenhärtbare Kunststoff-Füllstoffe auf Polyurethanbasis.

Füllstoffe zum Füllen von Kunststoff-Formulierungen zum Zwecke der Verbesserung der physikalischen Eigenschaften derselben sind hinreichend bekannt. Für dentale Werkstoffe beispielsweise werden seit langem neben anorganischen Füllstoffen, wie Quarz oder Gläser, organische Füllstoffe verwendet. Perlförmige Polymere und Copolymere auf Basis von Methylmethacrylat stellen hierbei einen weit verbreiteten Vertreter dar.

Ferner sind neben perlförmigen Polymeren aus der EP-B-0 270 915 beispielsweise Fällungspolymerisate zur Verwendung im Dentalbereich bekannt, die aus Acrylsäure und/oder Methacrylsäureestern gebildet werden.

Vorteilhaft an der Verwendung von organischen Füllstoffen ist u.a. die einfache Polierbarkeit der daraus hergestellten Compositematerialien, der günstige Preis im Vergleich zu ultrafein gemahlenen anorganischen Füllstoffen, die hohe Transparenz der erhaltenen Polymerisate sowie die aschefreie Verbrennbarkeit. Aufgrund der weiten Variabilität in der Zusammensetzung, die organische Füllstoffe aufweisen können, ist auch die gezielte Beeinflussung von Materialeigenschaften möglich, beispielsweise kann die Schlagzähigkeit von Dentalmaterialien durch die in der DE-A1-196 178 76 genannten Pfropf-Copolymerisate günstig beeinflußt werden.

Perlförmige Polymere und Copolymere auf Basis von Methylmethacrylat zeigen eine hohe Neigung zum Quellen. Diese ist erforderlich, um ein Anlösen der Füllstoffe durch Monomere zu ermöglichen, da durch die bei der Polymerisation erfolgende Bildung eines interpenetrierenden Netzwerks eine Anbindung der Füllstoffe an die Harzmatrix erst möglich wird. Durch diese Quellneigung wird aber ein stetiger Viskositätsanstieg der mit den Füllstoffen formulierten Massen verursacht. Bei Prothesenkunststoffen, die in der Regel das stark Iösungsvermittelnde Methylmethacrylat als Hauptbestandteil der Monomermatrix enthalten, bestimmt dieses Quellverhalten beispielsweise die Verarbeitungszeit in der Stopf-Press-Technik (Küvettentechnik). Die Messung des Quellverhaltens kann beispielsweise durch die in der internationalen Norm ISO 1567 beschriebene Messung der Verarbeitungszeit erfolgen, wobei ein Zeitrahmen von ca. 30 bis 60 Minuten als brauchbar gilt.

Die DE-C2-197 060 64 beschreibt plastisch aushärtbare Einkomponentenmassen auf Basis von PMMA-Perlen und höhermolekularen vernetzenden Methacrylaten. Obwohl Lagerstabilitäten von 6 Monaten beansprucht werden, tritt dennoch bereits bei leicht erhöhter Temperatur (36°C) innerhalb weniger Tage eine Verstrammung der Paste auf, welche auf den durch das Anlösen der PMMA-Perlen bedingten Viskositätsanstieg zurückzuführen ist.

Die aus der EP-B-0 270 915 bekannten Fällungspolymerisate werden zwar durch die in der dentalen Technologie üblichen (Meth)Acrylatmonomere aufgrund ihrer hohen Vernetzungsdichte nicht angelöst und zeigen daher auch keine Viskositätsänderungen im Verlauf der Lagerung, allerdings werden sie trotz vorhandener Restdoppelbindungen bei der Polymerisation nicht besonders gut in die Harzmatrix eingebaut, so dass die resultierenden Compositematerialien nur mäßige mechanische Eigenschaften aufweisen. Offensichtlich ist die Zugänglichkeit der Restdoppelbindungen nur bedingt gewährleistet.

Andere organische Füllstoffe, wie bei Raumtemperatur oder kryogen gemahlene Kunststoffpulver oder gefällte Polymerpulver zeigen ähnliche Probleme.

Die unter der Handelsbezeichnung "Coathylen" kommerziell erhältlichen Füllstoffe, basierend auf Polyethylen, Polypropylen, einem Ethylen-Acrylsäure-Acrylsäureester-Terpolymer oder Polyurethan, ergeben Compositematerialien mit sehr unzureichenden mechanischen Festigkeiten, da aufgrund fehlender Reaktionszentren kein Verbund zur Harzmatrix möglich ist.

Es besteht also erheblicher Bedarf an Füllstoffen auf organischer Basis, die in die Matrix einpolymerisiert werden können und deren Quellverhalten in den in der dentalen Technologie üblichen Monomermatrices so gering ist, dass sich Formulierungen mit einem stabilen Viskositätverhalten auch bei erhöhter Temperatur und längerer Lagerzeit realisieren lassen.

Aufgabe der vorliegenden Erfindung ist die Bereitstellung organischer Füllstoffe, die die oben angeführten Bedürfnisse befriedigen können.

Diese Aufgabe wird gelöst durch organische, vernetzte, reaktive und strahlenhärtbare Kunststoff-Füllstoffe auf Polyurethanbasis.

Die erfindungsgemäßen Fültstoffe weisen eine hohe Reaktivität auf, ohne dass sie einer starken Quellung in üblichen Dentalmonomeren unterliegen. Sie sind über ethylenische Doppelbindungen in Harzmatrices einpolymerisierbar, einfach und kostengünstig zu synthetisieren und ihre Eigenschaften können über Variation der Edukte in einem weiten Bereich eingestellt werden. Über die ungesättigten Funktionalitäten können sie strahlungsgehärtet werden und eignen sich daher besonders für den Einsatz im Dentalbereich, aber auch in anderen technischen Gebieten, wo die Eigenschaften der erfindungsgemäßen Füllstoffe von Vorteil sind.

Die erfindungsgemäßen Füllstoffe sind erhältlich durch Umsetzung von:
(A) 15 bis 35 Gew.-%, bevorzugt 20 bis 30 Gew.-% einer oder mehrerer strahlenhärtbarer Verbindungen auf (Meth)Acrylatbasis mit OH-Zahlen von 40 bis 700 mg KOH/g,
(B) 15 bis 40 Gew.-%, bevorzugt 20 bis 35 Gew.-% eines oder mehrerer Polyole mit einem Molekulargewicht von 500 bis 6000 g/mol,
(C) 0 bis 15 Gew.-%, bevorzugt 0 bis 10 Gew.-% eines oder mehrerer Polyole mit einem Molekulargewicht von unter 500 g/mol,
(D) 1 bis 10 Gew.-%, bevorzugt 1 bis 7 Gew.-% mindestens einer im Sinne der Isocyanatreaktion mono- und/oder difunktionellen Verbindung, die zusätzlich anionische Gruppen bzw. in anionische Gruppen umwandelbare funktionelle Gruppen enthält,
(E) 24 bis 69 Gew.-%, bevorzugt 34 bis 55 Gew.-% eines oder mehrerer Polyisocyanate,
   sowie anschließender Kettenverlängerung bzw. Vernetzung des resultierenden Produktes aus (A) bis (E) mit
(F) 0,5 bis 10 Gew.-%, bevorzugt 0,5 bis 5 Gew.-% bezogen auf die Gesamtmasse der Komponenten (A) bis (E) eines Gemisches einer oder mehrerer Diamine mit einem Polyamin der Funktionalität größer als 2,
wobei mindestens 30 Gew.-%, bevorzugt mindestens 50 Gew.-% der Komponente (F) aus Polyamin der Funktionalität größer als 2 besteht.

Strahlenhärtbare, aber wässrige Dispersionen ähnlicher Zusammensetzung sind aus der Lackindustrie bekannt. In der DE-A-195 25 489 bzw. DE-A-44 34 554 werden beispielsweise Polyester(meth)acrylaturethan-Dispersionen auf Basis von hydroxylgruppenhaltigen Polyester(meth)acrylatpräpolymeren beschrieben. Diese sind erhältlich durch Polyaddition von hydroxylgruppenhaltigen Polyester(meth)acrylatpräpolymeren und gegenüber Isocyanatgruppen reaktiven Verbindungen mit Polyisocyanaten sowie anschließender Umsetzung mit mehrfachfunktionellen Amines. Diese wässrigen Dispersionen bilden bei der Trocknung Filme und somit keine festen Partikel, die als Füllstoff verwendet werden können.

Überraschenderweise wurde jedoch gefunden, dass durch eine Umsetzung der oben genannten Komponenten (A) bis (E) mit anschließender Vernetzung durch die Komponente (F) die erfindungsgemäßen Füllstoffe nach Abziehen des Lösungsmittels erhalten werden. Besonders vorteilhaft ist hierbei, dass nach der Vernetzung mit (F) die Füllstoffe ohne zusätzliche Aufarbeitungsschritte erhalten werden können.

Bei der Komponente (A) handelt es sich um strahlenhärtbare Verbindungen auf (Meth)Acrylatbasis, die gemäß DIN 53 240 OH-Zahlen von 40 bis 700 mg KOH/g aufweisen. Die Bezeichnung (Meth)Acrylat wird in dieser Schrift stellvertretend für Methacrylat und/oder Acrylat eingesetzt.

Geeignete Komponenten (A) sind beispielsweise hydroxylgruppenhaltige Polyester(meth)acrylatpräpolymere, wie sie in der US-A-4 206 205, DE-OS-40 40 290, DE-OS-33 16 592, DE-OS-37 04 098 und in "UV & EB Curing Formulations for Printing inks Coatings and Paints", ed. R. Holman and P. Oldring, published by SITA Technology, London (England) 1988, S. 36 f. beschrieben werden. Alternativ können auch hydroxylgruppenhaltige Polyepoxy(meth)acrylatpräpolymere, die durch Umsetzung von Polyepoxiden mit (Meth)acrylsäure zugänglich sind, und/oder hydroxylgruppenhaltige Polyurethan(meth)acrylatpräpolymere eingesetzt werden. Besonders bevorzugt ist der Einsatz von hydroxylgruppenhaltigen Polyepoxy(meth)acrylatpräpolymeren, wie 2,2-Bis-4-(3-methacryloxy-2-hydroxypropyl)phenylpropan (Bis-GMA), 2,2-Bis-4-(3-acryloxy-2-hydroxypropyl)phenylpropan (Bis-GA) und von hydroxylgruppenhaltigen (Meth)Acrylatestern, wie Glycerinmono(meth)acrylat, Trimethylolpropanmono(meth)acrylat, oder Pentaerythritdi(meth)acrylat.

Polyole der Komponente (B) weisen ein Molekulargewicht von 500 bis 6000 g/mol auf und können in linearer oder schwach verzweigter Form vorliegen. Die Polyole können aus den bekannten chemischen Klassen polymerer Polyole, die in Polyurethansynthesen bzw. -formulierungen eingesetzt werden, entnommen sein. Beispielhaft zu nennen sind Polyester-, Polyesteramid-, Polyether-, Polythioether-, Polycarbonat-, Polyacetal-, Polyolefin-, Polysiloxan- und Poly(meth)acrylatpolyole.

Bei den Polyesterpolyolen handelt es sich um Umsetzungsprodukte von niedermolekularen Polyolen mit niedermolekularen Polycarbonsäuren.

Geeignete niedermolekulare Polyole bzw. Polyolgemische sind beispielsweise Ethylenglykol, Propylenglykol, 1,4-Butandiol, 1,6-Hexandiol, Diethylenglykol, Triethylenglykol, Neopentylglykol, 1,4-Bis(hydroxymethyl)-cyclohexan, Dipropylenglykol. Als höherfunktionelle Polyole, die anteilig mitverwendet werden können, um Verzweigungen in das Polyestermolekül einzubringen, sind beispielsweise Glycerin, Trimethylolpropan oder Pentaerythrit geeignet. Besonders bevorzugt sind 1,6-Hexandiol, Neopentylglykol und Trimethylolpropan.

Die niedermolekularen Polycarbonsäuren können aliphatischer, cycloaliphatischer, aromatischer und/oder heterocyclischer Natur sein. Anstelle der freien Polycarbonsäuren können auch entsprechende Polycarbonsäureanhydride oder Polycarbonsäureester mit niederen Alkoholen eingesetzt werden. Beispielhaft seien genannt: Bernsteinsäure, Adipinsäure, Sebacinsäure, Azelainsäure, Phthalsäure, Isophthalsäure, Phthalsäureanhydrid, Tetrahydrophthalsäureanhydrid, Glutarsäureanhydrid, Maleinsäure, Maleinsäureanhydrid, Fumarsäure, Terephthalsäuredimethylester. Besonders bevorzugt ist Adipinsäure.

Geeignete Polyesterpolyole sind unter der Bezeichnung "Desmophen" von der Firma Bayer erhältlich.

Polyester, die durch Polymerisation von Lactonen, wie Caprolacton, in Verbindung mit einem Polyol zugänglich sind, können ebenfalls eingesetzt werden. Polyesteramidpolyole können durch anteilige Verwendung von Aminoalkoholen, wie Ethanolamin, im Polyesterbildungsgemisch erhalten werden.

Einsetzbare Polyetherpolyole umfassen Produkte, die durch Polymerisation eines cyclischen Oxids, beispielsweise Ethylenoxid, Propylenoxid oder Tetrahydrofuran, oder durch Addition eines oder mehrerer dieser Oxide an polyfunktionelle Initiatoren, wie Wasser, Ethylenglykol, Propylenglykol, Diethylenglykol, Cyclohexandimethanol, Glycerin, Trimethylolpropan, Pentaerythrit oder Bisphenol A zugänglich sind. Besonders geeignete Polyetherpolyole sind Polyoxypropylendiole und -triole, Poly(oxyethylen-oxypropylen)diole und -triole, die durch gleichzeitige oder aufeinanderfolgende Addition von Ethylen- und Propylenoxid an geeignete Initiatoren erhalten werden, sowie Polytetramethylenetherglykole, die durch Polymerisation von Tetrahydrofuran entstehen.

Verwendbare Polythioetherpolyole sind u.a. Produkte, die durch Kondensation von Thiodiglykol alleine oder mit anderen Glykolen, Dicarbonsäuren, Formaldehyd, Aminoalkoholen oder Aminocarbonsäuren erhalten werden.

Einsetzbare Polycarbonatpolyole sind u.a. Produkte, die durch Umsetzung von Diolen, wie 1,3-Propandiol, 1,4-Butandiol, 1,6-Hexandiol, Diethylenglykol oder Tetraethylenglykol, mit Diarylcarbonaten, wie Diphenylcarbonat, oder mit Phosgen resultieren.

Geeignete Polyacetalpolyole sind Verbindungen, die durch Umsetzung von Glykolen wie Diethylenglykol, Triethylenglykol oder 1,6-Hexandiol mit Formaldehyd oder durch Polymerisation cyclischer Acetale hergestellt werden können.

Geeignete Polyolefinpolyole sind u.a. Butadienhomo- und -copolymere mit endständigen Hydroxylgruppen.

Geeignete Polysiloxanpolyole werden beispielsweise unter der Bezeichnung "Tegomer HSi" von der Fa. Goldschmidt vertrieben.

Geeignete Poly(meth)acrylatpolyole sind beispielsweise unter der Bezeichnung "Tegodiol" bei der Fa. Tego erhältlich.

Als Komponente (B) besonders bevorzugt sind Polyesterpolyole und Polycarbonatpolyole mit einem Molekulargewicht von 500 bis 6000 g/mol und insbesondere mit einem Molekulargewicht von 500 bis 3000 g/mol. Solche Verbindungen sind beispielsweise unter der Bezeichnung "Placcel" von der Firma Daicel kommerziell erhältlich.

Als Polyole der Komponente (C) mit einem Molekulargewicht von unter 500 g/mol sind folgende geeignet: aliphatische, cycloaliphatische, aromatische und/oder heterocyclische Verbindungen, wie sie im wesentlichen bei der Komponente (B) im Rahmen der Beschreibung zum Aufbau der Polyesterpolyole bereits genannt wurden. Besonders bevorzugte Polyole der Komponente (C) sind Neopentylglykol und Trimethylolpropan.

Bei der Komponente (D) handelt es sich beispielsweise um mindestens eine Hydroxycarbonsäure und/oder Aminocarbonsäure und/oder Aminosulfonsäure und/oder Hydroxysulfonsäure. Diese Verbindungen werden über die gegenüber den Isocyanaten der Komponente (E) reaktiven Amino- und/oder Hydroxygruppen in das Präpolymer, welches aus den Komponenten (A) bis (E) letztlich resultiert, eingebaut. Durch Neutralisation der Carboxylgruppen und/oder Sulfonsäuregruppen mit organischen und/oder anorganischen Basen erhalten die Verbindungen der Komponente (D) dispergierend wirkende Eigenschaften.

Exemplarisch seien als Vertreter der Komponente (D) genannt: Äpfelsäure, Glykolsäure, Glycin, Taurin, Aminocapronsäure, 2-Amino-ethylaminosulfonsäure. Zu den bevorzugten Vertretern der Komponente (D) gehören 2,2-Bis(hydroxymethyl)-alkanmonocarbonsäuren mit insgesamt 5 bis 8 Kohlenstoffatomen gemäß der allgemeinen Formel (1): 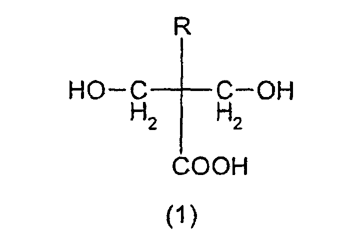 in welcher R für einen linearen, verzweigten oder cyclischen Alkylrest mit 1 bis 7 C-Atomen steht. Ganz besonders bevorzugte Aufbaukomponente (D) ist 2,2-Dimethylolpropionsäure.

Polyisocyanate, die als Komponente (E) geeignet sind, sind aliphatischer, cycloaliphatischer und/oder aromatischer Natur.

Beispiele für geeignete Polyisocyanate sind: 1,6-Hexamethylendiisocyanat (HDI), Tetramethylendiisocyanat, Isophorondiisocyanat, 4,4'-Dicyclohexylmethandiisocyanat, 1,4-Phenylendiisocyanat, 2,6- und 2,4-Toluoldiisocyanat, 1,5-Naphthylendiisocyanat, 2,4'- und 4,4'-Diphenylmethandiisocyanat. Es ist selbstverständlich auch möglich, die in der Polyurethanchemie an sich bekannten höherfunktionellen Polyisocyanate oder auch an sich bekannte modifizierte, beispielsweise Carbodiimidgruppen-, Allophanatgruppen-, Isocyanuratgruppen-, Urethangruppen- und/oder Biuretgruppenaufweisende Polyisocyanate einzusetzen bzw. anteilig mitzuverwenden. Besonders bevorzugte Isocyanate sind cycloaliphatische Isocyanate, wie Isophorondiisocyanat und 4,4'-Dicyclohexylmethandiisocyanat.

Die Komponenten (A) bis (E) werden in einem Reaktor vorgelegt oder einzeln zudosiert und unter wasserfreien Bedingungen in einem Temperaturbereich von 30°C bis 130°C zu einem NCO-haltigen Präpolymeren umgesetzt. Das Äquivalentverhältnis von Isocyanatgruppen zu gegenüber Isocyanatgruppen reaktiven Verbindungen beträgt 1,1 : 1 bis 3 : 1, bevorzugt 1,5 : 1 bis 2 : 1. Bei der Berechnung des Äquivalentverhältnisses werden Carboxylgruppen, die beispielsweise durch Mitverwendung von 2,2-Dimethylolpropionsäure in das Präpolymer eingebracht werden, nicht berücksichtigt. Die Isocyanatpolyadditionsreaktion kann in Gegenwart von in der Polyurethanchemie bekannten Katalysatoren, wie Organo-Zinn-Verbindungen, erfolgen. Weiterhin kann vor, während oder nach der Präpolymerherstellung ein organisches Lösungsmittel verwendet werden, um die Viskosität zu kontrollieren.

Geeignete Lösungsmittel sind beispielsweise Aceton, 2-Butanon, Tetrahydrofuran, Dioxan, Dimethylformamid, N-Methyl-2-pyrrolidon (NMP), Ethylacetat, Alkylether von Ethylen- und Propylenglykol und aromatische Kohlenwasserstoffe. Besonders bevorzugt ist der Einsatz von wassermischbaren, niedrigsiedenden Lösungsmitteln, wie Aceton, die aus den hergestellten Polyurethanpolyhamstoff-Dispersionen durch Destillation entfernt werden können:

Vor der Dispergierung des aus den Komponenten (A) bis (E) hergestellten Präpolymeren in Wasser und der Kettenverlängerung bzw. Vernetzung mit der Komponente (F) werden die im Präpolymeren vorliegenden potentiellen ionischen Gruppen beispielsweise durch Neutralisation in ionische Gruppen umgewandelt. Zur Neutralisation werden, insbesondere bei der Verwendung von Carboxylgruppen aufweisenden Aufbaukomponenten (D), bevorzugt tertiäre Amine eingesetzt. Derartige tertiäre Amine sind beispielsweise Triethylamin, Tri-n-butylamin, N-Methymorpholin, N,N-Dimethylethanolamin, N-Methylpiperidin, N-Methylpiperazin und Triethanolamin. Auch die Verwendung von anorganischen Basen, wie Natriumhydroxid oder Kaliumhydroxid als Neutralisationsmittel ist, wenn auch weniger bevorzugt, möglich. Es ist auch möglich, die Komponente (D) bereits in neutralisierter Form bei der Präpolymerherstellung einzusetzen.

Durch die Neutralisation der potentiellen ionischen Gruppen wird die Bildung stabiler wässriger Dispersionen gewährleistet. Im allgemeinen werden mindestens 80 %, bevorzugt jedoch 100 % der potentiellen ionischen Gruppen durch Neutralisation in ionische Gruppen überführt. Die Neutralisationsreaktion erfolgt dabei in der Regel bei Temperaturen von unter 100°C und bevorzugt im Temperaturbereich von 30 bis 80°C.

Die Überführung der neutralisierten NCO-haltigen Präpolymeren in wässrige Dispersionen erfolgt nach den in der Polyurethanchemie bekannten Methoden. Eine Möglichkeit besteht in der Zugabe des Dispergierwassers, welches die Komponente (F) enthält, zum Präpolymeren. Bei diesem Verfahren bildet das organische Präpolymer zunächst die kontinuierliche Phase. Bei weiterer Zugabe von Wasser findet eine Phasenumkehr statt und das Wasser wird zur kontinuierlichen Phase.

Bei einer anderen Möglichkeit der Dispergierung wird das neutralisierte Präpolymer zum Dispergierwasser gegeben. Die Komponente (F) kann dabei im Dispergierwasser vorgelegt sein oder alternativ erst nach der Dispergierung des Präpolymeren zugesetzt werden.

Der Dispergierschritt erfolgt bevorzugt in einem Temperaturbereich von 20 bis 40°C. Dabei kann die Dispergierbarkeit der Präpolymeren in Wasser durch den zusätzlichen Einsatz von externen Emulgatoren verbessert werden. Als exteme Emulgatoren sind beispielsweise Alkylsulfate, beispielsweise mit einer Kettenlänge von 8 bis 18 C-Atomen, Aryl- und Alkylethersulfate mit 8 bis 18 C-Atomen im hydrophoben Rest und 1 bis 40 Ethylenoxid-(EO-) bzw. Propylenoxid-(PO-)Einheiten geeignet.

Weiterhin können verwendet werden:
- Sulfonate, beispielsweise Alkylsulfonate mit 8 bis 18 C-Atomen, Alkylarylsulfonate mit 8 bis 18 C-Atomen, Ester und Halbester der Sulfobernsteinsäure mit einwertigen Alkoholen oder Alkylphenolen mit 4 bis 15 C-Atomen, wobei die Alkohole oder Alkylphenole auch mit 1 bis 40 EO-Einheiten ethoxyliert sein können,
- Alkali- und Ammoniumsalze von Carbonsäuren, besonders mit 8 bis 20 C-Atomen im Alkyl-, Aryl-, Alkaryl- oder Aralkylrest,
- Phosphorsäureteilester und deren Alkali- und Ammoniumsalze, beispielsweise Alkyl- und Alkarylphosphate mit 8 bis 20 C-Atomen im organischen Rest,
- Alkylether- bzw. Alkaryletherphosphate mit 8 bis 20 C-Atomen im Alkyl- bzw. Alkarylrest und 1 bis 40 EO-Einheiten,
- Alkylpolyglykolether mit 2 bis 40 EO-Einheiten und Alkylresten von 4 bis 20 C-Atomen,
- Alkylarylpolyglykolether mit 2 bis 40 EO-Einheiten und 8 bis 20 C-Atomen in den Alkyl- und Arylresten,
- Ethylenoxid/Propylenoxid (EO/PO)-Blockcopolymere mit 8 bis 40 EO- bzw. PO-Einheiten,
- Fettsäurepolyglykolester mit 6 bis 24 C-Atomen und 2 bis 40 EO-Einheiten und
- Alkylpolyglykoside.

Die Alkylreste können beispielsweise jeweils verzweigter, unverzweigter oder cyclischer Natur sein oder eine Mischung dieser Merkmale aufweisen.

Komponente (F) beschreibt Gemische aus einem oder mehreren Diaminen mit einem oder mehreren Polyaminen der Funktionalität größer als 2. Die Diamine führen zu einer Kettenverlängerung und damit zu einem Molekulargewichtsaufbau des Präpolymeren, während das Polyamin mit der Funktionalität größer als 2 eine Vernetzung der Molekülstruktur bewirkt. Die Umsetzung des Präpolymeren mit den Bestandteilen der Komponente (F) findet im wässrigen Medium statt. Daher weisen die Verbindungen der Komponente (F) bevorzugt im Vergleich zu Wasser eine höhere Reaktivität gegenüber Isocyanatgruppen auf. Die Menge an zu verwendender Komponente (F) hängt von den noch vorhandenen, nicht umgesetzten Isocyanatgruppen des Präpolymeren ab. Die Bestimmung des Isocyanatgehalts des Präpolymeren erfolgt gemäß DIN 53 185.

Als geeignete Diamine seien exemplarisch genannt: 1,2-Diaminoethan, 1,6-Diaminohexan, Piperazin, 2,5-Dimethylpiperazin, 1-Amino-3-aminoethyl-3,5,5-trimethylcyclohexan, 4,4'-Diaminodicyclohexylmethan, 1,4-Diaminocyclohexan und/oder 1,2-Propylendiamin. Als Kettenverlängerer geeignet sind auch Hydrazin, Aminosäurehydrazide, Bishydrazide und Bis-semicarbazide. Besonders bevorzugtes Diamin ist 1,2-Diaminoethan.

Beispiele für Polyamine mit einer Funktionalität größer als 2 sind Diethylentriamin, Triethylentetramin, Tetraethylenpentamin, Pentaethylenhexamin, Polyethylenimine und Melamin. Besonders bevorzugt ist der Einsatz von Diethylentriamin.

In der Lösung liegt der erfindungsgemäße Füllstoff beispielsweise in einer Konzentration von 20 bis 60 Gew.-%, insbesondere zwischen 25 und 45 Gew.-%, dispergiert vor. Der reine Füllstoff kann durch Abziehen des Lösungsmittels, beispielsweise mittels Vakuum oder auch durch Sprühtrocknung erhalten werden. Eine weitere Möglichkeit den Füllstoff aus der wässrigen Dispersion zu isolieren, besteht durch Koagulation der Dispersion mittels Salzzugabe oder durch Zugabe von polaren Lösungsmitteln. Besonders bevorzugt ist die Sprühtrocknung, da die erfindungsgemäßen Füllstoffe bei diesem Verfahren in einer kleinen Korngröße anfallen und direkt in weiteren Formulierungen verwendet werden können.

Die erfindungsgemäßen Füllstoffe eignen sich besonders zur Herstellung von Dentalmassen. Bevorzugt enthalten derartige Formulierungen folgende Komponenten:
(K1) 1 bis 40 Gew.-%, insbesondere 5 bis 30 Gew.-% erfindungsgemäßen Füllstoff,
(K2) 10 bis 98,9 Gew.-%, insbesondere 14 bis 94,9 Gew.-% ein oder mehrere ethylenisch ungesättigte polymerisierbare Monomere auf Basis di- oder mehrfunktioneller (Meth)acrylate,
(K3) 0 bis 75 Gew.-%, insbesondere 0 bis 50 Gew.-% übliche Füllstoffe,
(K4) 0,1 bis 3 Gew.-%, insbesondere 0,1 bis 2 Gew.-% Initiatoren und gegebenenfalls Aktivatoren,
(K5) 0 bis 10 Gew.-%, insbesondere 0 bis 5 Gew.-% Additive, gegebenenfalls Pigmente, Thixotropiehilfsmittel, Weichmacher.

Die aus den erfindungsgemäßen Füllstoffen formulierten Massen zeichnen sich durch besonders gute mechanische Eigenschaften sowie erheblichen Handlingsvorteilen aus. So sind sie aufgrund der guten Zähelastizität besonders hart, aber gleichzeitig flexibel. Die reaktiven (Meth)Acrlyatgruppen ermöglichen ein Einbinden der Füllstoffe in die Matrix der Formulierung. Durch die hohen Molmassen sind die Füllstoffe maximal biokompatibel und weisen keinerlei toxikologisch bedenkliche Eigenschaften auf. Bei geeigneter Wahl der Edukte verbrennen die Formulierungen nahezu aschefrei.

Als Komponente (K2) werden mindestens bifunktionelle Acrylsäure- und/oder Methacrylsäureester eingesetzt. Diese können monomere und polymere Acrylate und Methacrylate beinhalten. Vorteilhaft verwendet werden können beispielsweise die langkettigen Monomere der US-A-3 066 112 auf der Basis von Bisphenol-A und Glycidylmethacrylat oder deren durch Addition von Isocyanaten entstandene Derivate. Geeignet sind auch Verbindungen des Typs Bisphenol-Adiethyloxy(meth)acrylat und Bisphenol-A-dipropyloxy(meth)acrylat. Weiterhin Verwendung finden können die oligo-ethoxylierten und oligo-propoxylierten Bisphenol-A-diacryl- und -dimethacrylsäureester.

Gut geeignet sind weiter die Acrylsäure- und Methacrylsäureester mindestens bifunktioneller aliphatischer Alkohole, beispielsweise Triethylenglykol-di(meth)-acrylat, Ethylenglykol-di(meth)-acrylat, Hexandiol-di(meth)-acrylat und Trimethylolpropantri(meth)-acrylat.

Besonders geeignet sind auch die in der DE-C-28 16 823 genannten Diacryl- und Dimethacrylsäureester des Bis(hydroxymethyl)-tricyclo[5.2.1.0^{2,6}]-decans und die Diacryl- und Dimethacrylsäureester der mit 1 bis 3 Ethylenoxid- und/oder Propylenoxideinheiten verlängerten Verbindungen des Bis(hydroxymethyl)-tricyclo[5.2.1.0^{2,6}]-decans.

Gut geeignete Monomere sind auch die in der EP-A-0 235 826 beschriebenen Methacrylsäureester, beispielsweise Triglykolsäurebis[3(4)-methacryloxymethyl-8(9)-tricyclo[5.2.1.0^{2,6}]-decylmethylester].

Selbstverständlich können auch Gemische aus Monomeren und/oder aus hieraus hergestellten ungesättigten Polymeren verwendet werden.

Übliche Füllstoffe gemäß Komponente (K3) können anorganische Füllstoffe sein, beispielsweise Quarz, gemahlene Gläser, nicht wasserlösliche Fluoride, wie CaF₂, Kieselgele sowie Kieselsäure, insbesondere pyrogene Kieselsäure oder deren Granulate, sein. Zum besseren Einbau in die Polymermatrix kann es von Vorteil sein, diese Füllstoffe sowie gegebenenfalls röntgenopake Zusatzstoffe zu hydrophobieren. In einer bevorzugten Ausführungsform sind sämtliche eingesetzten anorganischen Füllstoffe silanisiert, vorzugsweise mit Trimethoxymethacryloxypropylsilan. Die Menge des eingesetzten Silans beträgt üblicherweise 0,5 bis 10 Gew.-%, bezogen auf anorganische Füllstoffe, bevorzugt 1 bis 6 Gew.-%, ganz besonders bevorzugt 2 bis 5 Gew.-%, bezogen auf anorganische Füllstoffe. Übliche Hydrophobierungsmittel sind Silane, beispielsweise Trimethoxymethacryloxypropylsitan. Die maximale mittlere Korngröße der anorganischen Füllstoffe beträgt vorzugsweise 15 µm, insbesondere 8 µm. Ganz besonders bevorzugt werden Füllstoffe mit einer mittleren Korngröße von < 3 µm eingesetzt.

Auch fluoridauslösende Füllstoffe, beispielsweise komplexe anorganische Fluoride aus der DE-A-44 45 266 sind verwendbar.

Desweitern können auch übliche perlförmige Polymere und Copolymere auf Basis von Methylmethacrylat eingesetzt werden, die beispielsweise bei der Fa. Röhm unter der Bezeichnung "Plexidon" oder "Plex" erhältlich sind.

Unter Initiatoren der Komponente (K4) sind Initiatorsysteme, die die radikalische Polymerisation der mindestens bifunktionellen Monomeren bewirken, beispielsweise Photoinitiatoren oder sogenannte Redoxinitiatorsysteme, aber auch thermische Initiatoren, zu verstehen.

Als Photoinitiatoren eignen sich beispielsweise α-Diketone, wie Campherchinon, in Verbindung mit sekundären und tertiären Aminen, oder Mono- und Bisacylphosphinoxide, wie 2,4,6-Trimethylbenzoyldiphenyl-phosphinoxid und Bis-(2,6-dichlorbenzoyl)-4-n-propylphenyl-phosphinoxid. Es eignen sich aber auch andere Verbindungen dieses Typs, wie sie in den europäischen Patentveröffentlichungsschriften EP-A-0 073 413, EP-A-0 007 508, EP-A-0 047 902, EP-A-0 057 474 und EP-A-0 184 095 beschrieben sind.

Als Redoxinitiatorsysteme eignen sich organische Peroxidverbindungen zusammen mit sogenannten Aktivatoren. Als organische Peroxidverbindungen kommen dabei insbesondere Verbindungen wie Lauroylperoxid, Benzoylperoxid sowie p-Chlorbenzoylperoxid und p-Methylbenzoylperoxid in Betracht.

Als Aktivatoren eignen sich beispielsweise tertiäre aromatische Amine, wie die aus der US-A-3 541 068 bekannten N,N-Bis-(hydroxyalkyl)-3,5-xylidine sowie die aus der DE-A-26 58 530 bekannten N,N-Bis-(hydroxyalkyl)-3,5-di-t-butylaniline, insbesondere N,N-Bis-(β-oxybutyl)-3,5-di-t-butylanilin sowie N,N-Bis-(hydroxyalkyl)-3,4,5-trimethylaniline.

Gut geeignete Aktivatoren sind auch die in der DE-B-14 95 520 beschriebenen Barbitursäuren und Barbitursäurederivate sowie die in der EP-A-0 059 451 beschriebenen Malonylsulfamide. Bevorzugte Malonylsulfamide sind 2,6-Dimethyl-4-isobutylmalonylsulfamid, 2,6-Diisobutyl-4-propylmalonylsulfamid, 2,6-Dibutyl-4-propylmalonylsulfamid, 2,6-Dimethyl-4-ethylmalonylsulfamid sowie 2,6-Dioctyl-4-isobutylmalonylsulfamid.

Zur weiteren Beschleunigung wird die Polymerisation hierbei vorzugsweise in Gegenwart von Schwermetallverbindungen und ionogenem Halogen oder Pseudohalogen durchgeführt. Als Schwermetall ist Kupfer, als Halogenid das Chloridion besonders geeignet Das Schwermetall wird geeigneterweise in Form löslicher organischer Verbindungen eingesetzt. Ebenso werden die Halogenid- und Pseudohalogenidionen geeigneterweise in Form von löslichen Salzen eingesetzt, beispielsweise genannt seien die löslichen Aminhydrochloride sowie quartäre Ammoniumchloridverbindungen.

Wenn die erfindungsgemäßen Dentalmassen als Komponente (K4) ein Redoxinitiatorsystem aus organischem Peroxid und Aktivator enthalten, so sind vorzugsweise Peroxid und Aktivator in räumlich voneinander getrennten Teilen der erfindungsgemäßen Dentalmasse vorhanden, die erst unmittelbar vor der Anwendung der erfindungsgemäßen Dentalmasse homogen miteinander vermischt werden. Enthält die erfindungsgemäße Dentalmasse als (K4) nebeneinander organisches Peroxid, Kupferverbindung, Halogenid und Malonylsulfamid und/oder Barbitursäure, so ist es insbesondere sinnvoll, dass organisches Peroxid, Malonylsulfamid und/oder Barbitursäure und die Kombination Kupferverbindung/Halogenid in drei räumlich voneinander getrennten Bestandteilen vorliegen. Beispielsweise können die Kombination Kupferverbindung/Halogenid, polymerisierbare Monomere sowie Füllstoffe zu einer Paste verknetet sein und die anderen Komponenten in oben beschriebener Weise jeweils mit einer geringen Menge an Füllstoffen oder insbesondere Thixotropie-Hilfsmitteln, wie silanisierter Kieselsäure, und einem Weichmacher, beispielsweise Phthalat, zu zwei separaten Pasten verknetet sein. Andererseits können die polymerisierbaren Monomeren auch zusammen mit organischem Peroxid und Füllem vorliegen.

Als Komponente (K5) können beispielsweise zur Erhöhung der Flexibilität der Massen lösliche organische Polymere eingesetzt werden. Geeignet sind beispielsweise Polyvinylacetat sowie die Copolymeren auf Basis Vinylchlorid/Vinylacetat, Vinylchlorid/Vinylisobutylether und Vinylacetat/Maleinsäuredibutylether. Gut geeignet als zusätzliche Weichmacher sind beispielsweise Dibutyl-, Dioctyl- und Dinonylphthalate oder -adipate sowie höhermolekulare Polyphthalsäureester und Adipinsäureester. Als Thixotropiehilfsmittel können neben pyrogenen Kieselsäuren auch modifizierte Schichtsilikate (Bentonite) oder organische Modifikatoren, beispielsweise auf Basis hydrierter Rizinusölderivate eingesetzt werden.

Dentalmaterialien, die die erfindungsgemäßen Füllstoffe enthalten, können beispielsweise Füllungsmaterialien, Zemente, provisorische Kronen- und Brückenmaterialien, Verblendkunststoffe, Prothesenmaterialien, kieferorthopädische Materialien, Kunststoffe zur Fissurenversiegelung, Modellierkunststoffe oder Modellkunststoffe sein.

Die erfindungsgemäßen Füllstoffe eignen sich auch für den Einsatz in Formulierungen zum Verkleben, Beschichten und Vergießen von Substraten, beispielsweise als Füllstoff für Spachtelmassen oder zur Verbesserungen der Eigenschaften von Kunststoffen im allgemeinen.

Die Erfindung wird nachfolgend durch Beispiele näher beschrieben, ohne dass sie durch diese beschränkt werden soll.

### Polyurethan-Füllstoff

### Herstellungsbeispiel 1

Ein mit einem Thermometer, Rückflußkühler und mechanischem Rührer ausgestatteter 2I-3-Halskolben wurde mit 200 g Bis-GMA (Komponente A), 40,2 g Dimethylolpropionsäure (Komponente D), 23,1 g 1,6-Hexandiol (Komponente C), 195,8 g eines Polyesterpolyols (Komponente B) hergestellt aus Terephthalsäure/Neopentylglykol mit einem Molekulargewicht von 1000 g/mol, 420 g Aceton, 333 g Isophorondiisocyanat (Komponente E) und 0,1 g Dibutylzinndilaurat beschickt. Das Reaktionsgemisch wurde 5 Stunden auf 60°C erwärmt, bis der Isocyanat-Gehalt auf 3,9 % gefallen war. Der Reaktor wurde auf 20°C gekühlt und das Gemisch mit 27,2 g Triethylamin neutralisiert.

Die erhaltene Präpolymerlösung wird bei 23°C unter Rühren in 1152 g entionisiertem Wasser dispergiert und nachfolgend durch Zugabe von 7,0 g Ethylendiamin (Komponente F) und 8,0 g Diethylentriamin (Komponente F) vernetzt. Nach zwanzig Stunden hatte die Dispersion einen pH-Wert von 7,7 und einen Feststoffgehalt von 34,5%.

Die Dispersion wurde in dünner Schicht im Trockenschrank bei 40°C getrocknet. Das erhaltene Granulat wurde durch Mahlen auf eine Korngrößenverteilung von 50% < 60 µm, 99% < 200 µm gebracht.

### Herstellungsbeispiel 2

Ein mit einem Thermometer, Rückflußkühler und mechanischem Rührer ausgestatteter 2I-3-Halskolben wurde mit 200 g Bis-GMA (Komponente A), 40,2 g Dimethylolpropionsäure (Komponente D), 214,5 g ethoxyliertes Bisphenol-A (Komponente B) mit dem Molekulargewicht 550, 356 g Tetrahydrofuran, 333 g Isophorondiisocyanat (Komponente E) und 0,1 g Dibutylzinndilaurat beschickt. Das Reaktionsgemisch wurde 5 Stunden auf 60°C erwärmt, bis der Isocyanat-Gehalt auf 3,5 % gefallen war. Der Reaktor wurde auf 20°C gekühlt und das Gemisch mit 27,2 g Triethylamin neutralisiert.

Die erhaltene Präpolymerlösung wird bei 23°C unter Rühren in 1296 g entionisiertem Wasser dispergiert und nachfolgend durch Zugabe von 9 g Ethylendiamin (Komponente F) und 7,8 g Diethylentriamin (Komponente F) vernetzt. Nach zwanzig Stunden hatte die Dispersion einen pH-Wert von 7,9 und einen Feststoffgehalt von 33,5%.

Die Dispersion wurde in dünner Schicht im Trockenschrank bei 40°C getrocknet. Das erhaltene Granulat wurde durch Mahlen auf eine Korngrößenverteilung von 50% < 60 µm, 99% < 200 µm gebracht.

### Zahntechnische Modellmaterialien

### Herstellungsbeispiel 3

### 1. Herstellung einer Monomerlösung

Die in der nachfolgenden Tabelle aufgelisteten Bestandteile werden in einem geeigneten Rotlichtraum in einem Erlenmeyerkolben gerührt, bis eine homogene Lösung erhalten wird.

| | |
|---|---|
| 74,27 g | Bis-GMA stab. mit 200 ppm Hydrochinonmonomethylether (HQME) |
| 18,57 g | Bis(hydroxymethyl)-tricyclo[5.2.1.0^{2,6}]-decandiacrylat stab. mit 100 ppm HQME und 180 ppm Jonol |
| 0,40 g | Bis-(2,6-dichlorbenzoyl)-4-n-propylphenyl-phosphinoxid |
| 6,76 g | Poly(phthalsäure-1,6-hexandiolester) mit einer Viskosität von 1200 bis 1300 mPas |

### 2. Herstellung der Pasten

Unter Verwendung eines Laborkneters werden daraus die nachfolgend beschriebenen Pasten hergestellt. Die Füllstoffzugaben erfolgen sukzessive, nach jeder Teilzugabe wird bis zur Homogenität unter reduziertem Druck (200 mbar) geknetet. Die Knetzeiten betragen zwischen 6 h and 9 h. Die Mengenangaben erfolgen in Gewichtsprozent.

| | Com-1 | Com-2 | Com-3 | Com-4 | Com-5 |
|---|---|---|---|---|---|
| Monomerlösung | 83% | 69% | 69% | 67% | 72% |
| Fällungspolymerisat^{#} | 17%* | 13% | 13% | 13% | 13% |
| PU-Füllstoff 1 | | 18% | | | |
| PU-Füllstoff 2 | | | 18% | | |
| Plex-6690-F | | | | | 15% |
| Coathylen TB 2957 | | | | 20% | |

| | | | | | |
|---|---|---|---|---|---|
| * ein höherer Füllgrad ist nicht zu erreichen | | | | | |
| ^{#} nach Herstellungsbeispiel 3 der EP-0 270 915 | | | | | |

Die erhaltenen Pasten werden 1 Tag bei 50°C gelagert. Hierbei erfolgt nochmals bei allen Pasten ein gewisser Viskositätsanstieg.

Die so hergestellten Pasten weisen nach der Aushärtung (Lichtpolymerisationsgerät Visio beta vario, Fa. ESPE) die nachfolgend aufgelisteten mechanischen Eigenschaften auf:

| | Com-1 | Com-2 | Com-3 | Com-4 | Com-5 |
|---|---|---|---|---|---|
| Biegefestigkeit [MPa] | 48 | 77 | 69 | 35 | 27 |
| E-Modul[MPa] | 1300 | 2200 | 1980 | 990 | 1020 |
| Kugeldruckhärte[MPa] | 79 | 100 | 106 | 43 | 41 |

Werden die erhaltenen Pasten bei unterschiedlichen Temperaturen eingelagert, so lassen sich folgende Beobachtungen erzielen:

| Lagerdauer | Temperatur | Com-1 | Com-2 | Com-3 | Com-4 | Com-5 |
|---|---|---|---|---|---|---|
| 1 Woche | 4°C | i.O. | i.O. | i.O. | i.O. | i.O. |
| | 23°C | i.O. | i.O. | i.O. | i.O. | i.O. |
| | 36°C | i.O. | i.O. | i.O. | i.O. | Verstrammt |
| 1 Monat | 4°C | i.O. | i.O. | i.O. | i.O. | i.O. |
| | 23°C | i.O. | i.O. | i.O. | i.O. | Verstrammt |
| | 36°C | i.O. | i.O. | i.O. | i.O. | --- |
| 6 Monate | 4°C | i.O. | i.O. | i.O. | i.O. | Verstrammt |
| | 23°C | i.O. | i.O. | i.O. | i.O. | -- |
| | 36°C | i.O. | i.O. | i.O. | Klebrig, gummiartig | -- |
| 12 Monate | 4°C | i.O. | i.O. | i.O. | i.O. | -- |
| | 23°C | i.O. | i.O. | i.O. | Klebrig, gummiartig | -- |
| | 36°C | i.O. | i.O. | i.O. | -- | -- |

### Provisorische Kronen und Brückenmaterialien

### Herstellunqsbeispiel 4

### 1. Herstellung einer Katalysatorpaste

Die in der Tabelle aufgelisteten Bestandteile werden in einem Kneter vermischt, bis eine homogene Paste mit einer Viskosität von 78 Pas erhalten wird. Die Knetzeiten betragen zwischen 4 h und 7,5 h.

| | |
|---|---|
| 38,9 g | Acetyliertes Bisphenol-A mit einem Ethoxylierungsgrad von 4 |
| 5 g | Poly(phthalsäure-1,6-hexandioiester) mit einer Viskosität von 1200 bis 1300 mPas |
| 51 g | SrAIB-Silikatglas(d₅₀ = 10 µm, silanisiert mit 1% 3-Methacroylpropoxytrimethoxysilan) |
| 4,1 g | Di(4-methylbenzoyl)peroxid |
| 1 g | Pyrogene Kieselsäure |

### 2. Herstellung der Basispasten

Die in Tabelle aufgelisteten Bestandteile werden in einem Kneter vermischt, bis eine homogene Paste mit einer Viskosität zwischen 8-12 Pas erhalten wird. Die Knetzeiten betragen zwischen 2,5 h und 4,5 h. Die Mengenangaben erfolgen in Gewichtsprozent.

| | PKB-1 | PKB-2 |
|---|---|---|
| 2,2-Bis-(4-di(ethoxy)phenyl)propandimethacrylat | 46,5 % | 46,5 % |
| 7,7,9-Trimethyl-4,13-dioxo-3,14-dioxa-5,12-diazahexadecan-1,16-dioxydimethacrylat | 31 % | 31 % |
| SrAIB-Silikatglas(d₅₀ = 10 µm, silanisiert mit 1% 3-Methacroylpropoxytrimethoxysilan) | 18 % | 13 % |
| PU-Füllstoff nach Beispiel 2 | 0 % | 5 % |
| Pyrogene Kieselsäure | 3 % | 3 % |
| N,N-Bis-(2-hydroxyethyl)-4-methylanilin | 1,5 % | 1,5 % |

Die so hergestellten Pasten werden im Verhältnis 4:1 (Basis:Katalysator) vermischt. Die ausgehärtetn Formulierungen weisen die nachfolgend aufgelisteten mechanischen Eigenschaften auf:

| | PKB-1 | PKB-2 |
|---|---|---|
| Biegefestigkeit [MPa] | 73 | 71 |
| E-Modul [MPa] | 1300 | 1420 |
| Schlagzähigkeit [mJ/mm²] | 3,23 | 5,18 |

## Patentansprüche

1. Füllstoff für Kunststoff-Formulierungen auf Polyurethanbasis, erhältlich durch Umsetzung von:
(A) 15 bis 35 Gew.-% einer oder mehrerer strahlenhärtbarer Verbindungen auf (Meth)Acrylatbasis mit OH-Zahlen von 40 bis 700 mg KOH/g
(B) 15 bis 40 Gew.-% eines oder mehrerer Polyole mit einem Molekulargewicht von 500 bis 6000 g/mol
(C) 0 bis 15 Gew.-% eines oder mehrerer Polyole mit einem Molekulargewicht von unter 500 g/mol
(D) 1 bis 10 Gew.-% mindestens einer im Sinne der Isocyanatreaktion mono- undloder difunktionellen Verbindung, die zusätzlich anionische Gruppen bzw. in anionische Gruppen umwandelbare funktionelle Gruppen enthält
(E) 24 bis 69 Gew.-% eines oder mehrerer Polyisocyanate
sowie anschließender Kettenverlängerung bzw. Vernetzung des resultierenden Produktes aus (A) bis (E) mit
(F) 0,5 bis 10 Gew.-%, bezogen auf die Gesamtmasse der Komponenten (A) bis (E), eines Gemisches mindestens eines Diamins mit einem Polyamin der Funktionalität größer als 2,
wobei mindestens 30 Gew.-%, bevorzugt 50 Gew.-% der Komponente (F) aus Polyamin der Funktionalität größer als 2 besteht.

2. Füllstoff nach Anspruch 1, wobei die Komponenten (A) bis (F) wie folgt definiert sind:
(A) eine oder mehrere der folgenden Verbindungen: hydroxylgruppenhaltige Polyester(meth)acrylatpräpolymere, hydroxylgruppenhaltige Polyepoxy(meth)acrylatpräpolymere, hydroxylgruppenhaltige Polyurethan(meth)acrylatpräpolymere, hydroxylgruppenhaltige (Meth)Acrylatester,
(B) eine oder mehrere der folgenden Verbindungen: Polyester-, Polyesteramide-, Polyether-, Polythioether-, Polycarbonat-, Polyacetal-, Polyolefin-, Polysiloxan- und Poly(meth)acrylatpolyole,
(C) eine oder mehrere der folgenden Verbindungen: Ethylenglykol, Propylenglykol, 1,4-Butandiol, 1,6-Hexandiol, Diethylenglykol, Triethylenglykol, Neopentylglykol, 1,4-Bis(hydroxymethyl)-cyclohexan, Dipropylenglykol, Glycerin, Trimethylolpropan oder Pentaerythrit,
(D) eine oder mehrere der folgenden Verbindungen: Äpfelsäure, Glykolsäure, Glycin, Taurin, Aminocapronsäure, 2-Amino-ethylaminosulfonsäure, 2,2-Bis(hydroxymethyl)-alkanmonocarbonsäuren mit insgesamt 5 bis 8 Kohlenstoffatomen gemäß der allgemeinen Formel (1): 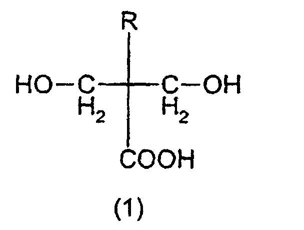 in weicher R für einen linearen, verzweigten oder cyclischen Alkylrest mit 1 bis 7 C-Atomen steht,
(E) eine oder mehrere der folgenden Verbindungen: 1,6-Hexamethylendiisocyanat, Tetramethylendiisocyanat, Isophorondiisocyanat, 4,4'-Dicyclohexylmethandiisocyanat, 1,4-Phenylendiisocyanat, 2,6- und 2,4-Toluoldiisocyanat, 1,5-Naphthylendiisocyanat, 2,4'- und 4,4'-Diphenylmethandiisocyanat, höherfunktionellen Polyisocyanate oder modifizierte Isocyanate wie Carbodiimidgruppen-, Allophanatgruppen-, lsocyanuratgruppen- undloder Biuretgruppen-aufweisenden Polyisocyanate,
(F) eine oder mehrere der folgenden Verbindungen: 1,2-Diaminoethan, 1,6-Diaminohexan, Piperazin, 2,5-Dimethylpiperazin, 1-Amino-3-aminoethyl-3,5,5-trimethylcyclohexan, 4,4'-Diaminodicyclohexylmethan, 1,4-Diaminocyclohexan, 1,2-Propylendiamin, Hydrazin, Aminosäurehydrazide, Bishydrazide, Bis-semicarbazide und Polyamine mit einer Funktionalität größer als 2.

3. Füllstoff nach einem der Ansprüche 1 oder 2, wobei die Komponenten (A) bis (F) wie folgt definiert sind:
(A) eine oder mehrere der folgenden Verbindungen: 2,2-Bis-4-(3-methacryloxy-2-hydroxypropyl)phenylpropan, 2,2-Bis-4-(3-acryloxy-2-hydroxypropyl)phenylpropan, Glycerinmonoacrylat, Glycerinmonomethacrylat, Trimethylolpropanmonoacrylat, Trimethylolpropanmonomethacrylat, Pentaerythritdiacrylat, Pentaerythritdimethacrylat,
(B) eine oder mehrere der folgenden Verbindungen: Polyester- und Polycarbonatdiole,
(C) eine oder mehrere der folgenden Verbindungen: Neopentylglykol, Trimethylolpropan, 1,6-Hexandiol,
(D) 2,2-Dimethylolpropionsäure,
(E) Isophorondiisocyanat und/oder 4,4'-Dicyclohexylmethandiisocyanat,
(F) als Diamin: 1,2-Diaminoethan; als Polyamin mit einer Funktionalität größer als 2: Diethylentriamin.

4. Verwendung der Füllstoffe nach einem der Ansprüche 1 bis 3 zur Herstellung von Dentalmassen.

5. Verwendung der Füllstoffe nach einem der Ansprüche 1 bis 3 zur Herstellung von Füllungsmaterialien, Zementen, provisorischen Kronen- und Brückenmaterialien, Verblendkunststoffen, Prothesenmaterialien, kieferorthopädischen Materialien, Kunststoffen zur Fissurenversiegelung, Modellierkunststoffen und Modellkunststoffen.

6. Verwendung der Füllstoffe nach einem der Ansprüche 1 bis 3 in Formulierungen zum Beschichten, Verkleben oder Vergießen von Substraten.

7. Verfahren zur Herstellung von Füllstoffen für Kunststoff-Formulierungen auf Polyurethanbasis, wobei folgende Schritte umfasst sind:
(1) Umsetzung einer Mischung von:
(A) 15 bis 35 Gew.-% einer oder mehrerer strahlenhärtbarer Verbindungen auf (Meth)Acrylatbasis mit OH-Zahlen von 40 bis 700 mg KOH/g;
(B) 15 bis 40 Gew.-% eines oder mehrerer Polyole mit einem Molekulargewicht von 500 bis 6000 g/mol;
(C) 0 bis 15 Gew.-% eines oder mehrerer Polyole mit einem Molekulargewicht von unter 500 g/mol;
(D) 1 bis 10 Gew.-% mindestens einer im Sinne der Isocyanatreaktion mono- und/oder difunktionellen Verbindung, die zusätzlich anionische Gruppen bzw. in anionische Gruppen umwandelbare funktionelle Gruppen enthält;
(E) 24 bis 69 Gew.-% eines oder mehrerer Polyisocyanate;
zu Präpolymeren;
(2) Neutralisation der in den Präpolymeren vorliegenden potentiellen ionischen Gruppen;
(3) Dispergierung in Wasser sowie Kettenverlängerung bzw. Vernetzung mit:
(F) 0,5 bis 10 Gew.-%, bezogen auf die Gesamtmasse der Komponenten (A) bis (E), eines Gemisches mindestens eines Diamins mit einem Polyamin der Funktionalität größer als 2,
wobei mindestens 30 Gew.-% der Komponente (F) aus Polyamin der Funktionalität größer als 2 besteht;
(4) Aufarbeitung.

8. Füllstoffe nach den Ansprüchen 1 bis 3 enthaltende Massen, umfassend:
(K1) 1 bis 40 Gew.-% Füllstoff nach mindestens einem der Ansprüche 1 bis 3,
(K2) 10 bis 98,9 Gew.-% ein oder mehrere ethylenisch ungesättigte polymerisierbare Monomere auf Basis di- oder mehrfunktioneller (Meth)acrylate,
(K3) 0 bis 75 Gew.-% übliche Füllstoffe,
(K4) 0,1 bis 3 Gew.-% Initiatoren und gegebenenfalls Aktivatoren,
(K5) 0 bis 10 Gew.-% Additive, gegebenenfalls Pigmente, Thixotropiehilfsmittel, Weichmacher.

## Claims

1. Filler for plastics formulations based on polyurethane, obtainable by reaction of:
(A) 15 to 35 wt.% of one or more radiation-curing (meth)acrylate-based compounds with OH numbers of 40 to 700 mg KOH/g
(B) 15 to 40 wt.% of one or more polyols with a molecular weight of 500 to 6,000 g/mol
(C) 0 to 15 wt.% of one or more polyols with a molecular weight of less than 500 g/mol
(D) I to 10 wt.% of at least one compound which is mono- and/or difunctional in the sense of the isocyanate reaction, which additionally contains anionic groups or functional groups which can be converted into anionic groups
(E) 24 to 69 wt.% of one or more polyisocyanates,
and subsequent chain lengthening or crosslinking of the resulting product from (A) to (E) with
(F) 0.5 to 10 wt.%, relative to the total weight of components (A) to (E), of a mixture of at least one diamine with a polyamine of functionality greater than 2,
at least 30 wt.%, preferably 50 wt.% of component (F) comprising polyamine of functionality greater than 2.

2. Filler according to claim 1, wherein components (A) to (F) are defined as follows:
(A) one or more of the following compounds: polyester-(meth)acrylate prepolymer containing hydroxyl groups, polyepoxy(meth)acrylate prepolymer containing hydroxyl groups, polyurethane-(meth)acrylate prepolymer containing hydroxyl groups and (meth)acrylate ester containing hydroxyl groups,
(B) one or more of the following compounds: polyester-, polyester-amide-, polyether-, polythioether-, polycarbonate-, polyacetal-, polyolefin-, polysiloxane- and poly(meth)acrylate-polyols,
(C) one or more of the following compounds: ethylene glycol, propylene glycol, 1,4-butanediol, 1,6-hexanediol, diethylene glycol, triethylene glycol, neopentylglycol, 1,4-bis(hydroxymethyl)-cyclohexane, dipropylene glycol, glycerol, trimethylolpropane or pentaerythritol.
(D) one or more of the following compounds: malic acid, glycolic acid, glycine, taurine, aminocaproic acid, 2-amino-ethylaminosulphonic acid, 2,2-bis(hydroxymethyl)-alkanemonocarboxylic acids with a total of 5 to 8 carbon atoms according to the general formula (1): 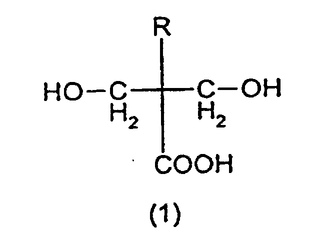 in which R represents a linear, branched or cyclic alkyl radical with I to 7 C atoms,
(E) one or more of the following compounds: 1,6-hexamethylene diisocyanate, tetramethylene diisocyanate, isophorone diisocyanate, 4,4'-dicyclohexylmethane diisocyanate, 1,4-phenylene diisocyanate, 2,6- and 2,4-toluene diisocyanate, 1,5-naphthylene diisocyanate, 2,4'- and 4,4'-diphenylmethane diisocyanate, polyisocyanates of higher functionality or modified isocyanates, such as polyisocyanates containing carbodiimide groups, allophanate groups, isocyanurate groups and/or biuret groups,
(F) one or more of the following compounds: 1,2-diaminoethane, 1,6-diaminohexane, piperazine, 2,5-dimethylpiperazine, 1-amino-3-aminoethyl-3,5,5-trimethylcyclohexane, 4,4'-diaminodicyclohexylmethane, 1,4-diaminocyclohexane, 1,2-propylenediamine, hydrazine, amino acid hydrazides, bishydrazides. bis-semicarbazides and polyamines with a functionality greater than 2.

3. Filler according to one of claims I or 2, wherein components (A) to (F) are defined as follows:
(A) one or more of the following compounds: 2,2-bis-4-(3-methacryloxy-2-hydroxypropyl)phenylpropane, 2,2-bis-4-(3-acyyloxy-2-hydroxypropyl)phenylpropane, glycerol monoacrylate. glycerol monomethacrylate, trimethylolpropane monoacrylate, trimethylolpropane monomethacrylate, pentaerythritol diacrylate, pentaerythritol dimethacrylate.
(B) one or more of the following compounds: polyester- and polycarbonate-diols,
(C) one or more of the following compounds: neopentylglycol, trimethylolpropane, 1,6-hexanediol,
(D) 2,2-dimethylolpropionic acid,
(E) isophorone diisocyanate and/or 4,4'-dicyclohexylmethane diisocyanate,
(F) as diamine: 1,2-diaminoethane; as polyamine with a functionality greater than 2: diethylenetriamine.

4. Use of the fillers according to one of claims 1 to 3 for the preparation of dental compositions.

5. Use of the fillers according to one of claims 1 to 3 for the preparation of filling materials, cements, temporary crown and bridge materials, veneer plastics, prosthesis materials, orthodontic materials, plastics for sealing fissures, modelling plastics and model plastics.

6. Use of the fillers according to one of claims 1 to 3 in formulations for coating, gluing or embedding substrates.

7. Process for the preparation of fillers for plastics formulations based on polyurethane, comprising the following steps:
(1) reaction of a mixture of:
(A) 15 to 35 wt.% of one or more radiation-curing (meth)acrylate-based compounds with OH numbers of 40 to 700 mg KOH/g;
(B) 15 to 40 wt.% of one or more polyols with a molecular weight of 500 to 6,000 g/mol;
(C) 0 to 15 wt.% of one or more polyols with a molecular weight of less than 500 g/mol;
(D) 1 to 10 wt.% of at least one compound which is mono- and/or difunctional in the sense of the isocyanate reaction, which additionally contains anionic groups or functional groups which can be converted into anionic groups;
(E) 24 to 69 wt.% of one or more polyisocyanates,
to give prepolymers;
(2) neutralization of the potential ionic groups present in the prepolymers;
(3) dispersing in water and chain lengthening or crosslinking with:
(F) 0.5 to 10 wt.%, relative to the total composition of components (A) to (E), of a mixture of at least one diamine with a polyamine of functionality greater than 2;
at least 30 wt.% of component (F) comprising polyamine of functionality greater than 2;
(4) working up.

8. Fillers according to claims 1 to 3 containing compositions comprising:
(C1) 1 to 40 wt.% of filler according to at least one of claims 1 to 3,
(C2) 10 to 98.8 wt.% of one or more ethylenically unsaturated polymerizable monomers based on di- or polyfunctional (meth)acrylates.
(C3) 0 to 75 wt.% of conventional fillers.
(C4) 0.1 to 3 wt.% of initiators and, where appropriate, activators.
(C5) 0 to 10 wt.% of additives, where appropriate pigments, thixotropy auxiliaries and plasticizers.

## Revendications

1. Charge pour formulations de matière plastique à base de polyuréthanne, pouvant être obtenue pas réaction de :
(A) 15 à 35% en poids d'un ou de plusieurs composés durcissables par irradiation, à base de (méth)acrylate avec un indice OH allant de 40 à 700 mg KOH/g ;
(B) 15 à 40% en poids d'un ou de plusieurs polyols avec un poids moléculaire allant de 500 à 6000 g/mole ;
(C) 0 à 15% en poids d'un ou de plusieurs polyols avec un poids moléculaire inférieur à 500 g/mole ;
(D) 1 à 10% en poids d'au moins un composé mono- et/ou difonctionnel dans le sens d'une réaction avec les isocyanates, qui contient en outre, des radicaux anioniques ou des radicaux fonctionnels pouvant être convertis en radicaux anioniques ;
(E) 24 à 69% en poids d'un ou de plusieurs polyisocyanates,
ainsi que ensuite, l'allongement de chaîne ou la réticulation du produits résultant de (A) à (E) avec
(F) 0,5 à 10% en poids, sur base de la masse totale des composants (A) à (E), d'un mélange d'au moins une diamine avec une polyamine de fonctionnalité supérieure à 2,
où au moins 30% en poids, de préférence au moins 50% en poids du composant (F) consiste en la polyamine de fonctionnalité supérieure à 2.

2. Charge selon la revendication 1, où les composants (A) à (F) sont définis comme suit :
(A) un ou plusieurs des composés suivants : prépolymères de polyester-(méth)acrylate contenant des radicaux hydroxyle, prépolymères de polyépoxy-(méth)acrylate contenant des radicaux hydroxyle, prépolymères de polyuréthanne-(méth)acrylate contenant des radicaux hydroxyle, esters de (méth)acrylate contenant des radicaux hydroxyle ;
(B) un ou plusieurs des composés suivants : polyester-, polyesteramide-, polyéther-, polythioéther-, polycarbonate-, polyacétal-, polyoléfine-, polysiloxane- et poly(méth)acrylate-polyol ;
(C) un ou plusieurs des composés suivants : éthylèneglycol, propylèneglycol, 2,4-butanediol, 1,6-hexanediol, diéthylèneglycol, triéthylèneglycol, néopentylglycol, 1,4-bis(hydroxyméthyl)cyclohexane, dipropylèneglycol, glycérine, triméthylolpropane ou pentaérythritol ;
(D) un ou plusieurs des composés suivants : acide malique, acide glycolique, glycine, taurine, acide aminocaproïque, acide 2-aminoéthylaminosulfonique, acides 2,2-bis(hydroxyméthyl)alcanemonocarboxyliques avec au total, 5 à 8 atomes de carbone selon la formule générale (1) : 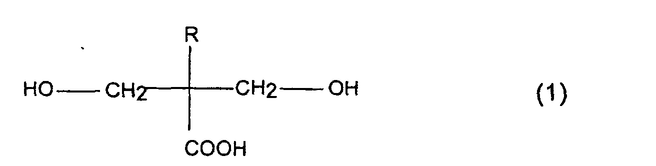 dans laquelle R représente un reste alkyle linéaire, ramifié ou cyclique ayant 1 à 7 atomes C,
(E) un ou plusieurs des composés suivants : 1,6-hexaméthylènediisocyanate, tétraméthylènediisocyanate, isophoronediisocyanate, 4,4'-dicyclohexylméthanediisocyanate, 1,4-phénylènediisocyanate, 2,6- et 2,4-toluylènediisocyanate, 1,5-naphtylènediisocyanate, 2,4'- et 4,4'-diphénylméthanediisocyanate, des polyisocyantes à fonctionnalité supérieure ou des isocyanates modifiés comme des polyisocyanates présentant des radicaux carbodiimide, allophanate, isocyanurate et/ou biuret ;
(F) un ou plusieurs des composés suivants : 1,2-diaminoéthane, 1,6-diaminohexane, pipérazine, 2,5-diméthylpipérazine, 1-amine-3-aminoéthyl-3,5,5-triméthylcyclohexane, 4,4'-diaminodicyclohexylméthane, 1,4-diaminocyclohexane, 1,2-propylènediamine, hydrazine, hydrazides d'acide aminé, bishydrazides, bis-semicarbazides et polyamines avec une fonctionnalité supérieure à 2.

3. Charge selon l'une quelconque des revendications 1 ou 2, où les composants (A) à (F) sont définis de la manière suivante :
(A) un ou plusieurs des composés suivants : 2,2-bis-4-(3-méthacryloxy-2-hydroxypropyl)phénylpropane, 2,2-bis-4-(3-acryloxy-2-hydroxypropyl)phénylpropane, monoacrylate de glycérine, monométhacrylate de glycérine, monoacrylate de triméthylolpropane, monométhacrylate de triméthylolpropane, diacrylate de pentaérythritol, diméthacrylate de pentaérythritol ;
(B) un ou plusieurs des composés suivants : polyester- et polycarbonate-polyols ;
(C) un ou plusieurs des composés suivants : néopentylglycol, triméthylolpropane, 1,6-hexanediol ;
(D) acide 2,2-diméthylolpropionique ;
(E) isophoronediisocyanate et/ou 4,4'-dicyclohexylméthanediisocyanate ;
(F) comme diamine : 1,2-diaminoéthane, comme polyamine avec une fonctionnalité supérieure à 2 : diéthylènetriamine.

4. Utilisation de la charge selon l'une quelconque des revendications 1 à 3, pour la préparation de masses dentaires.

5. Utilisation de la charge selon l'une quelconque des revendications 1 à 3, pour la préparation de matériaux de remplissage, ciments, matériaux de couronne et de bridge provisoires, matières plastiques de revêtement, matériaux de prothèse, matériaux orthopédiques pour la mâchoire, matières plastiques pour masquage des fissures, matière plastique de modelage et matières plastiques des modèles.

6. Utilisation de la charge selon l'une quelconque des revendications 1 à 3, pour la préparation de revêtements, colles ou masquages de substrats.

7. Procédé de préparation de charges pour formulation de matière plastique à base de polyuréthanne, qui comprend les étapes suivantes :
(1) réaction d'un mélange de :
(A) 15 à 35% en poids d'un ou de plusieurs composés durcissables par irradiation, à base de (méth)acrylate avec un indice OH allant de 40 à 700 mg KOH/g ;
(B) 15 à 40% en poids d'un ou de plusieurs polyols avec un poids moléculaire allant de 500 à 6000 g/mole ;
(C) 0 à 15% en poids d'un ou de plusieurs polyols avec un poids moléculaire inférieur à 500 g/mole ;
(D) 1 à 10% en poids d'au moins un composé mono- et/ou difonctionnel dans le sens d'une réaction avec les isocyanates, qui contient en outre, des radicaux anioniques ou des radicaux fonctionnels pouvant être convertis en radicaux anioniques ;
(E) 24 à 69% en poids d'un ou de plusieurs polyisocyanates,
en un prépolymères ;
(2) neutralisation des radicaux ioniques potentiels présents dans le prépolymère ;
(3) dispersion dans l'eau ainsi que l'allongement de chaîne ou la réticulation avec
(F) 0,5 à 10% en poids, sur base de la masse totale des composants (A) à (E), d'un mélange d'au moins une diamine avec une polyamine de fonctionnalité supérieure à 2
où au moins 30% en poids des composants (F) consistent en des polyamines de fonctionnalité supérieure à 2 ;
(4) traitement.

8. Masses contenant un charge selon l'une quelconque des revendications 1 à 3, comprenant :
(K1) 1 à 40% en poids de charge selon l'une quelconque des revendications 1 à 3,
(K2) 10 à 98 9% en poids d'un ou de plusieurs monomères polymérisables éthyléniquement insaturés à base de (méth)acrylate di- ou polyfonctionnel,
(K3) 0 à 75% en poids de charges usuelles,
(K4) 0,1 à 3% en poids d'initiateurs et le cas échéant, d'activateurs,
(K5) 0 à 10% en poids d'additifs, le cas échéant de pigments, d'auxiliaires de thixotropie, de plastifiants.
